# EUROPEAN PATENT SPECIFICATION

(11) **EP 0 922 051 B1**
(45) Date of publication and mention of the grant of the patent: **09.05.2001**
(21) Application number: 97935730.8
(22) Date of filing: 07.08.1997
(51) Int. Cl.: C07H 17/08, C07H 17/00

(54) **PROCESS FOR THE PRODUCTION OF SALTS OF ERYTHROMYCIN, ROXITHROMYCIN, CLARITHROMYCIN AND AZITHROMYCIN**
VERFAHREN ZUR HERSTELLUNG VON SALZEN VON ERYTHROMICYN, ROXITHROMICYN, CLARYTHROMICYN UND AZITHROMYCIN
PROCEDE POUR LA PRODUCTION DE SELS D'ERYTHROMYCINE, DE ROXITHROMYCINE, DE CLARITHROMYCINE ET D'AZITHROMYCINE

(30) Priority: 07.08.1996 IE 960568
(43) Date of publication of application: 16.06.1999
(73) Proprietor: RUSSINSKY LIMITED, Cork (IE)
(72) Inventor: SCHICKANEDER, Helmut, South Terrace, Cork (IE); NIKOLOPOULOS, Aggelos, Off Wellington Road, Cork (IE)
(74) Representative: O'Brien, John Augustine
(86) International application number: IE9700056
(87) International publication number: WO9805674

(56) References cited:
- EP-A- 0 057 489
- EP-A- 0 096 013
- WO-A-96/19489
- FR-A- 2 561 105
- US-A- 4 563 443
- US-A- 4 826 820

## Description

The present invention relates to a new process for the production of antibiotic and mucolytic salts of erythromycin, roxithromycin, clarithromycin, azithromycin, and propionyl esters of erythromycin.

The therapeutical properties of such salts has been partly described in EP 0057489A. The process for producing these salts as described in EP 0057489A is characterised in that the reaction is carried out in an organic solvent and in the presence of water in an amount not greater than 20%. Salts produced by this process include impurities and side products such as decomposition compounds of erythromycin (8, 9-anhydrous-hemiketal) and oxidation compounds of N-acetylcysteine (N,N-diacetyl-cystine). Thus, the quality of salts produced by the process is not sufficient for pharmaceutical preparations.

WO 96/19489A discloses a process for the preparation of a salt between acetylcystein and an antibiotic selected from roxithromycin, clarithromycin and azithromycin, comprising homogenising the antibiotic and the N-acetylcystein under inert conditions and adding water at atmospheric pressure.

FR 2561105A discloses salts of erythromycin prepared in the presence of an organic solvent with a maximum water content of 20%.

EP 0096013A discloses the salt of erythromycin with DL-α- mercaptopropionyl-glycine and a diastereomeric form thereof. The preparation of the compounds in the presence of an organic solvent is described.

All the prior art processes involve the use of organic solvents.

It is an object of the present invention to provide a process for the production of the above-mentioned antibiotic and mucolytic salts produced without any solvent, in large quantities in almost quantitative yields without increased amounts of impurities and side products.

### Statements of Invention

According to the invention there is provided a process for preparing a compound of the formula

R⁺X⁻

wherein X is a radical selected from a group of compounds comprising sulphur containing acids, and wherein R is a radical selected from:-
erythromycin, clarithromycin, roxithromycin, azithromycin, and a propionyl ester of erythromycin,
the process comprising reacting X with R in the presence of water, the process, including the water addition, being carried out in an inert atmosphere.

The inert atmosphere typically comprises nitrogen gas.

In one embodiment of the invention X and R are initially homogenised prior to the addition of water. Preferably X and R are homogenised for a period of from 1 to 2 hours. Ideally X and R are homogenised at a temperature of from 10 to 20°C.

In one embodiment of the invention the initial homogenisation of X and R is carried out in an inert atmosphere. Ideally water is added to the reactants in an inert atmosphere.

In a particularly preferred embodiment of the invention the reaction mixture including water is homogenised during the reaction step.

Preferably the reaction mixture is homogenised for a period of from 1 to 2 hours. Most preferably the reaction mixture is homogenised at a temperature of from 10°C to 30°C.

In one embodiment of the invention the reactants are milled to break up any particulate matter. Preferably the reactants are milled during a homogenisation step.

X may be a radical selected from:-
acetylcysteine, carboxymethylcysteine and thiazolidin-carboxylic acid.

In a further embodiment of the invention the desired compound is dried under vacuum at 20 to 50°C.

The invention also provides a compound R⁺X⁻ as defined above whenever produced by the process of the invention.

### Detailed Description of the Invention

### Example

Salts of erythromycin-2-propionate with N-acetylcysteine.

327.5 Kg of erythromycin-2-propionate and 76.28 Kg of N-acetylcysteine were charged under nitrogen into an INOX vacuum dryer, Model IUT. The reactants were homogenised and milled under nitrogen for 1-2 hours at 10-20°C. To this mixture, 150 L of purified water was added under nitrogen and the resultant aqueous mixture was further homogenised and milled for 1-2 hours at 10-30°C under nitrogen to produce a suspension of the desired salt. The suspension was then dried under vacuum at 20-50°C. A yield of between 96 and 98.2% (430-440 Kg) was obtained.

The carboxymethylcysteine, N-acetylcysteine and thiazolidin-carboxylic acid salts of erythromycin, clarithromycin, roxithromycin and azithromycin are produced in a similar manner to that described in the above example. A similar process may also be used for the production of carboxymethylcysteine and thiazolidin-carboxylic acid salts of erythromycin-2-propionate. Pages 1/2 and 2/2 of the annex show the molecular structures of different macrolide antibiotic salts i.e. roxithromycin, erythromycin, erythromycin-2-propionic ester, clarithromycin and azithromycin acetylcysteinat.

The process described above allows the described compounds to be produced, without any solvent, in large quantities in almost quantitative yields without increased amounts of impurities and side products.

The reactors sold under the Trade Mark INOX are ideally suited for carrying out the process of the invention. Such reactors are described in US4674692 and US4506838. In particular, the INOX models IST, IHT and IUT are particularly suited for carrying out the process of the invention. The reactors are essentially vacuum-dryer reactors having a paddle rotating concentrically about an axis of the reactor which homogenises and agitates the contents of the reactor. The reactor may include a chopping and milling tool rotatably mounted on one of the paddles for rotation together therewith. The rotational action of both the paddle and the tool ensure complete mixing and milling of the contents of the reactor, ensuring that all lumps of material are uniformly broken down and homogenised with the other materials in the reactor. In some of the models mentioned above, the drying chamber may be rotated. The model IUT mentioned above may include a granulating unit comprising a series of spray nozzles located at a back end of a paddle ensuring an even distribution of liquid to be granulated.

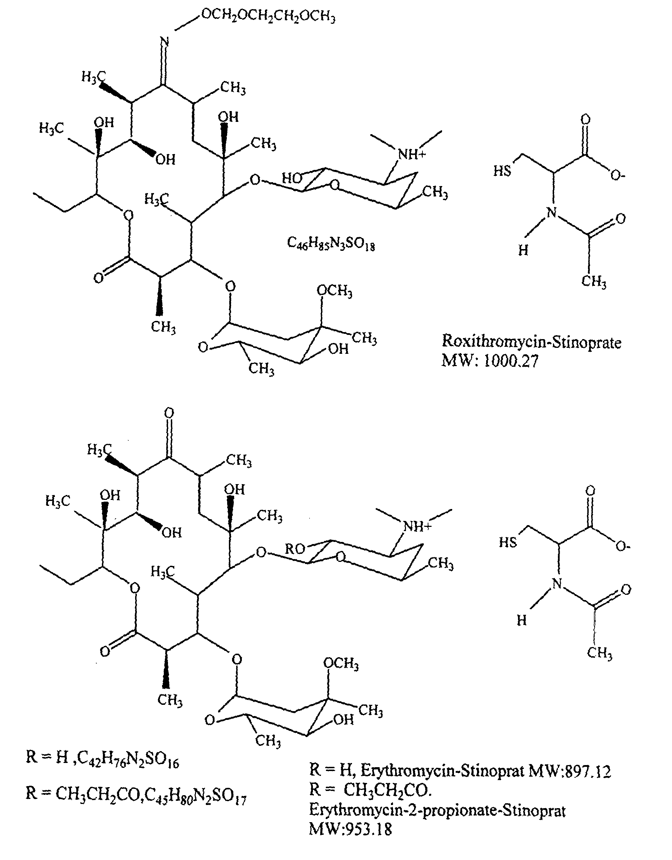

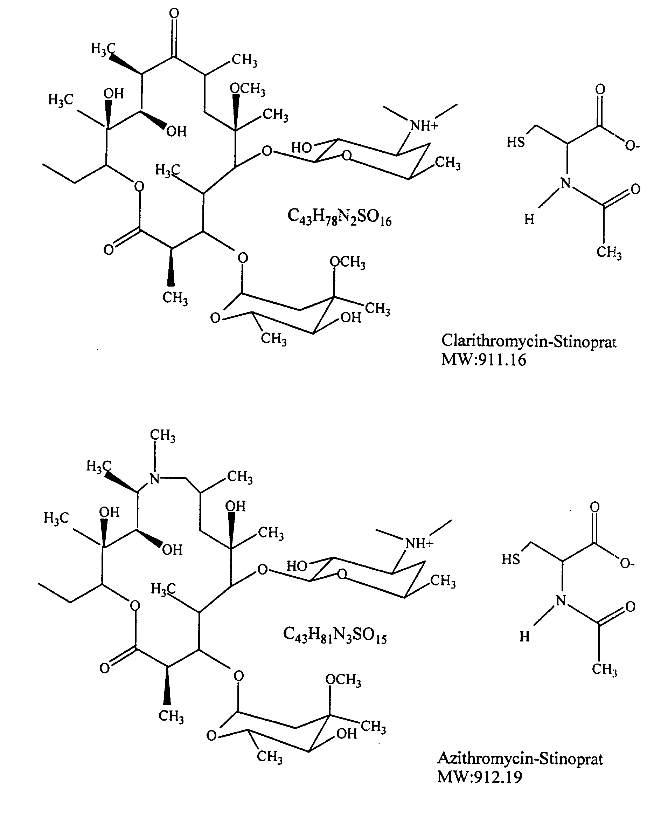

## Claims

1. A process for preparing a compound of the formula
R⁺X⁻
wherein X is a radical selected from a group of compounds comprising sulphur containing acids,
and wherein R is a radical selected from:-
erythromycin, clarithromycin, roxithromycin, azithromycin, and a propionyl ester of erythromycin,
the process comprising reacting X with R in the presence of water, the process, including the water addition, being carried out in an inert atmosphere.

2. A process as claimed in claim 1 wherein the inert atmosphere comprises nitrogen gas.

3. A process as claimed in claim 1 wherein X and R are initially homogenised prior to the addition of water.

4. A process as claimed in claim 3 wherein X and R are homogenised for a period of from 1 to 2 hours.

5. A process as claimed in claim 3 or 4 wherein X and R are homogenised at a temperature of from 10 to 20°C.

6. A process as claimed in any of claims 3 to 5 wherein the initial homogenisation of X and R is carried out in an inert atmosphere.

7. A process as claimed in any preceding claim wherein the reaction mixture including water is homogenised during the reaction step.

8. A process as claimed in claim 7 wherein the reaction mixture is homogenised for a period of from 1 to 2 hours.

9. A process as claimed in claim 7 or 8 wherein the reaction mixture is homogenised at a temperature of from 10°C to 30°C.

10. A process as claimed in any preceding claim wherein the desired compound is dried under vacuum.

11. A process as claimed in claim 10 wherein the desired compound is dried under vacuum at 20 to 50°C.

12. A process as claimed in any preceding claim wherein X is a radical selected from:-
acetylcysteine, carboxymethylcysteine and thiazolidin-carboxylic acid.

## Patentansprüche

1. Verfahren zur Herstellung einer Verbindung der Formel
R⁺X⁻
worin X ein Radikal ist, das aus einer Gruppe von Verbindungen ausgewählt wird, die Schwefel enthaltende Säuren umfasst,
und worin R ein Radikal ist, das aus Folgendem ausgewählt wird:
Erythromycin, Clarithromycin, Roxithromycin, Azithromycin und einem Propionylester von Erythromycin,
wobei das Verfahren das zur Reaktion bringen von X mit R in der Gegenwart von Wasser umfasst, wobei das Verfahren, einschließlich der Wasserzugabe, in einer inerten Atmosphäre durchgeführt wird.

2. Verfahren nach Anspruch 1, worin die inerte Atmosphäre Stickstoffgas umfasst.

3. Verfahren nach Anspruch 1, worin X und R vor der Zugabe von Wasser anfänglich homogenisiert werden.

4. Verfahren nach Anspruch 3, worin X und R für eine Zeitdauer von 1 bis 2 Stunden homogenisiert werden.

5. Verfahren nach Ansprüchen 3 und 4, worin X und R bei einer Temperatur von 10°C bis 20°C homogenisiert werden.

6. Verfahren nach einem der Ansprüche 3 bis 5, worin die anfängliche Homogenisierung von X und R in einer inerten Atmosphäre durchgeführt wird.

7. Verfahren nach einem der vorangehenden Ansprüche, worin das Reaktionsgemisch, einschließlich Wasser, während des Reaktionsschrittes homogenisiert wird.

8. Verfahren nach Anspruch 7, worin das Reaktionsgemisch für eine Zeitdauer von 1 bis 2 Stunden homogenisiert wird.

9. Verfahren nach Anspruch 7 oder 8, worin das Reaktionsgemisch bei einer Temperatur von 10°C bis 30°C homogenisiert wird.

10. Verfahren nach einem der vorangehenden Ansprüche, worin die gewünschte Verbindung unter Vakuum getrocknet wird.

11. Verfahren nach Anspruch 10, worin die gewünschte Verbindung unter Vakuum bei 20°C bis 50°C getrocknet wird.

12. Verfahren nach einem der vorangehenden Ansprüche, worin X ein Radikal ist, das aus Folgendem ausgewählt wird:
Acetylcystein, Carboxymethylcystein und Thiazolidin-Carbonsäure.

## Revendications

1. Procédé pour préparer un composé de la formule
R⁺X⁻
dans lequel X est un radical sélectionné d'un groupe de composés comprenant des acides contenant du soufre,
et dans lequel R est un radical sélectionné de :
l'érythromycine, la clarithromycine, la roxithromycine, l'azithromycine, et d'un ester propionylique de l'érythromycine,
le procédé comprenant faire réagir X avec R en présence d'eau, le procédé, l'addition d'eau incluse, étant effectué dans une atmosphère inerte.

2. Procédé tel que revendiqué dans la revendication 1 dans lequel l'atmosphère inerte comprend du gaz azote.

3. Procédé tel que revendiqué dans la revendication 1 dans lequel X et R sont initialement homogénéisés avant l'addition d'eau.

4. Procédé tel que revendiqué dans la revendication 3 dans lequel X et R sont homogénéisés pendant une période de 1 à 2 heures.

5. Procédé tel que revendiqué dans la revendication 3 ou 4 dans lequel X et R sont homogénéisés à une température de 10 à 20°C.

6. Procédé tel que revendiqué dans l'une quelconque des revendications 3 à 5 dans lequel l'homogénéisation initiale de X et R est effectuée dans une atmosphère inerte.

7. Procédé tel que revendiqué dans l'une quelconque des revendications précédentes dans lequel le mélange de réaction, l'eau incluse, est homogénéisé pendant l'étape de réaction.

8. Procédé tel que revendiqué dans la revendication 7 dans lequel le mélange de réaction est homogénéisé pendant une période de 1 à 2 heures.

9. Procédé tel que revendiqué dans la revendication 7 ou 8 dans lequel le mélange de réaction est homogénéisé à une température de 10°C à 30°C.

10. Procédé tel que revendiqué dans l'une quelconque des revendications précédentes dans lequel le composé désiré est séché sous vide.

11. Procédé tel que revendiqué dans la revendication 10 dans lequel le composé désiré est séché sous vide à 20 jusqu'à 50°C.

12. Procédé tel que revendiqué dans l'une quelconque des revendications précédentes dans lequel X est un radical sélectionné de :
l'acétylcystéine, la carboxyméthylcystéine et l'acide thiazolidine carboxylique.
